(19) Europäisches Patentamt
European Patent Office
Office européen des brevets

(11) Publication number: **0 171 968**
**A1**

(12) # EUROPEAN PATENT APPLICATION

(21) Application number: 85305407.0

(22) Date of filing: 30.07.85

(51) Int. Cl.[4]: A 61 K 31/685
//C07F9/65

(30) Priority: 02.08.84 JP 163581/84

(43) Date of publication of application:
19.02.86 Bulletin 86/8

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Nojima, Shoshichi
723, 24-7, Nakano 2-chome
Nakano-ku Tokyo 164(JP)

(72) Inventor: Nomura, Hiroaki
9-15, Higashikanmaki 2-chome
Takatsuki Osaka 569(JP)

(72) Inventor: Tsushima, Susumu
1-705, 3 Momoyamadai 4-chome
Suita Osaka 565(JP)

(74) Representative: Laredo, Jack Joseph et al,
Elkington and Fife High Holborn House 52/54 High
Holborn
London, WC1V 6SH(GB)

(54) **Use of a phospholipid derivative.**

(57) This invention provides a method for inhibiting the proliferation of tumors in a warm-blooded animal or prolonging the life span of said animal, which comprises administering to said animal an effective amount of a compound of the formula

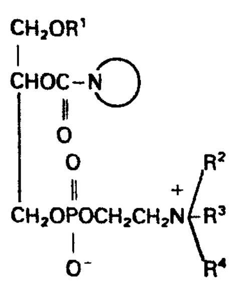

wherein $R^1$ is a $C_{14-19}$ alkyl group; $R^2$, $R^3$ and $R^4$ are independently $C_{1-2}$ alkyl or 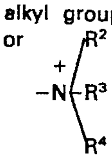 represents a nitrogen-containing five- or six-membered heterocyclic ring; and 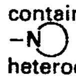 is a nitrogen-containing four- to six-membered heterocyclic group, or a pharmaceutically acceptable salt thereof, for the manufacture of a medicament for inibiting the proliferation of tumors in a warm-blooded animal or prolonging the life span of said animal.

EP 0 171 968 A1

SPECIFICATION

Use of A Phospholipid Derivative

This invention relates to use of a phospholipid derivative. More particularly, this invention relates to use of a compound of the formula

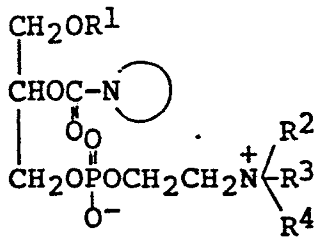

(I)

wherein $R^1$ is a $C_{14-19}$ alkyl group; $R^2$, $R^3$ and $R^4$ are independently $C_{1-2}$ alkyl or 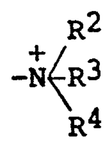 represents a nitrogen-containing five- or six-membered heterocyclic ring; and 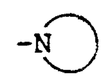 is a nitrogen-containing four- to six-membered heterocyclic group, or a pharmaceutically acceptable salt thereof, for the manufacture of a medicament for inhibiting the proliferation of tumors in a warm-blooded animal or prolonging the life span of said animal.

The compounds represented by the formula (I),

from the standpoint of their chemical structures, are analogs of the platelet activating factor (PAF). PAF is a mediator released from basophils in the antigen-antibody reactions between an antigen and IgE antibody, and has been identified structurally by Hanahan et al. [J. Biol. Chem., 254, 9355 (1979)] as a glycerophospholipid having an octadecyl (or hexadecyl) group through the ether linkage at the 1-position of glycerol and an acetyl group at the 2-position i.e. 1-O-octadecyl-2-O-acetyl-sn-glycero-3-phosphocholine. The factor (as chemically synthesized), when injected in an extremely small amount into animals, not only causes activation of platelets and neutrophils but also exhibits, for example, vessel-permeability increasing, smooth muscle constricting and blood-pressure lowering actions.

The said compounds, normally, through intravenous injection in not less than 1.5 μg/head, can bring about typical anaphylactic shock in rabbits, and thus acute toxic death of rabbits can be observed. As mentioned above, PAF shows extremely strong toxicity, and therefore, it is considered unsuitable to use it itself as a drug. In addition, PAF has been known to be readily susceptible to hydrolysis and inactivation by acetyl hydrolase existing in living organisms, and it is also generally noticed that PAF lacks the long duration of action as a drug.

In the Japanese Unexamined Patent Publication No. 2636/1980, also, there have been disclosed compounds possessing anticancer activity, whereby almost all of the compounds disclosed in the said gazette, nevertheless, have an acyl group as $R^1$. The compounds having an acyl group as $R^1$ in vivo, undergo readily enzymatic hydrolysis and inactivation, and exhibit anticancer activity being markedly inferior in potency and duration to the corresponding compounds having alkyl as $R^1$. Although only one compound (No. 3) having alkyl as $R^1$ has been

disclosed in the said gazzette, the said compound has carbamoyl at the 2-position of the glycerol skeleton. The compounds having a carbamoyl group at the 2-position of the glycerol skeleton, like PAF, are found to exhibit severe side effects, such as platelet-activating, neutrophil-activating, tissue-impairing, vessel-permeability increasing and blood-pressure lowering actions, and such side effects place restrictions on their utilization as a pharmaceutical.

Because PAF and the compounds as specifically described in the Japanese Unexamined Patent Publication No. 2636/1980 all, in vivo, undergo readily enzymatic hydrolysis and inactivation or exhibit potent toxicity by themselves, there have been encountered difficulties in employing them as an antitumor agent. The present inventors, with a specific view to the search for an antitumor agent which is resistant to enzymes existing in vivo and also exhibits extremely low toxicity, conducted intensive research, and as a result, succeeded in preparing the antitumor agents which possess potent antitumor activities inclusive of macrophage activating and tumor necrotizing actions while exhibiting outstandingly reduced side effects such as platelet activating and blood pressure lowering actions, which has led to the completion of the present invention.

With reference to the above formula (I), the $C_{14-19}$ alkyl group represented by $R^1$ includes straight-chain alkyl groups such as tetradecyl, pentadecyl, hexadecyl, heptadecyl, octadecyl and nonadecyl, and branched-chain alkyl groups such as 3,7,11-trimethyldodecyl, whereby straight-chain alkyl groups having 16 to 18 carbon atoms among others, are preferred.

The $C_{1-2}$ alkyl group represented by $R^2$, $R^3$ and $R^4$ includes alkyl groups such as methyl and ethyl.

The nitrogen-containing five-membered heterocyclic group represented by the group $-\overset{+}{N}\begin{matrix}R^2\\R^3\\R^4\end{matrix}$ includes groups, such as thiazolio, oxazolio and N-methylpyrrolidinio, while the nitrogen-containing six-membered heterocyclic group includes groups, such as pyridinio, N-methylpiperazinio, N-methylmorpholinio and N-methylpiperidinio.

The nitrogen-containing four- to six-membered heterocyclic group represented by the group 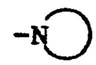 includes groups, such as azetidino, pyrrolidino, piperidino, morpholino, piperazino and thiomorpholino.

Incidentally, the compound (I) in some instances exists in the form of salts, such as pharmaceutically acceptable salts represented by the formulae:

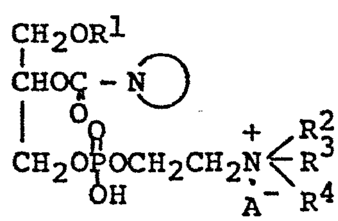

(Ia)

[wherein $A^-$ is an anion, such as chlorine, bromine, iodine

and tosyl ions; the others are as defined hereinbefore] and

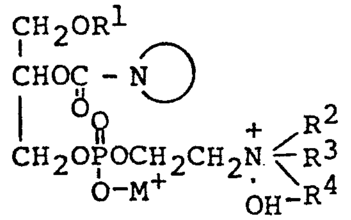
(Ib)

[wherein $M^+$ is an alkali metal (e.g., Na, K) ion or alkaline earth metal (e.g., Ca, Mg) ion]. The conversion of (I) into (Ia) or (Ib) and the conversion of (Ia) or (Ib) into (I) can be readily carried out by the per se known methods.

The compounds which are used as the antitumor agent according to the present invention are specifically disclosed by the following compounds:

1-O-Octadecyl-2-O-(pyrrolidinocarbonyl)glycero-3-phosphocholine, 1-O-octadecyl-2-O-(morpholinocarbonyl)-glycero-3-phosphocholine, 1-O-octadecyl-2-O-(piperidinocarbonyl)glycero-3-phosphocholine, 1-O-octadecyl-2-O-(azetidinocarbonyl)glycero-3-phosphocholine, 3-octadecyloxy-2-(morpholinocarbonyloxy)propyl 2-(N-methylmorpholinio)-ethyl phosphate, 3-octadecyloxy-2-(pyrrolidinocarbonyloxy)-propyl 2-(N-methylpyrrolidinio)ethyl phosphate, 3-octa-decyloxy-2-(azetidinocarbonyloxy)propyl 2-(N-methylpyrrolidinio)-ethyl phosphate, 3-octadecyloxy-2-(morpholinocarbonyloxy)-propyl 2-pyridinioethyl phosphate, 3-octadecyloxy-2-(morpholinocarbonyloxy)propyl 2-thiazolioethyl phosphate, 3-octadecyloxy-2-(pyrrolidinocarbonyloxy)propyl 2-thiazolioethyl phosphate, 3-hexadecyloxy-2-(morpholinocarbonyloxy)-propyl 2-thiazolioethyl phosphate, 3-hexadecyloxy-2-(morpholinocarbonyloxy)propyl 2-pyridinioethyl phosphate, 3-octadecyloxy-2-(pyrrolidinocarbonyloxy)propyl 2-pyridinioethyl phosphate and 3-octadecyloxy-2-(piperidinocarbonyloxy)-propyl 2-pyridinioethyl phosphate.

The compounds (I) and their salts can be readily produced by the method as described in the Japanese Unexamined

Patent Publication No. 192825/1983 or those analogous thereto.

With regard to the compound (I), there exists two kinds of the stereoisomers of R and S configurations, and their individual stereoisomers or a mixture thereof may be used.

The antitumor agent according to the present invention possesses excellent antitumor activities, with extremely reduced side effects, and can be administered per se or as a suitable pharmaceutical composition, as a safe antitumor agent, to cancer-bearing warm-blooded animals.

The compound (I) is well absorbed even on oral administration, and can be safely administered parenterally or orally. While the dosage level varies depending upon the conditions of the disease to be treated as well as the route of administration, in the case of administration to cancer-bearing warm-blooded animals as an antitumor agent, the compound as an injectable solution is normally administered at a single dose of preferably about 0.03 to 50 mg/kg as the compound (I), preferably once to twice per day according to the conditions. When the compound is administered through other parenteral routes of administration and orally, the dosage levels are suitably determined in accordance with the case of an injectable solution.

The pharmaceutical composition which is to be used as an antitumor agent contains an effective amount of the compound (I) or its salt, as an active ingredient, and a pharmaceutically acceptable carrier or excipient. Such a composition is provided as a dosage form suitable for oral or parenteral administration.

Thus, the composition for oral administration, for example, may be in solid or liquid dosage forms which are specifically exemplified by tablets (inclusive of

sugar-coated tablets and film-coated tablets), pills, granules, powders, capsules (inclusive of soft capsules), syrups, emulsions, suspensions, etc. Such compositions are manufactured by the per se known processes, and contain a carrier or excipient conventionally used in the art. For example, the carrier and excipient for tablets include lactose, starch, sucrose, magnesium stearate, etc.

The composition for parenteral administration for example, may be in dosage forms, such as injectable solutions and suppositories, wherein the injectable solutions include such injectable solutions for intravenous and intramuscular administration. Such injectable solutions are prepared by the per se known processes, namely by dissolving, suspending or emulsifying the compound (I) in a sterile aqueous or oily solution. The aqueous solution for injections includes, for example, physiological saline and isotonic solutions, which, if necessary, may be used in combination with a suitable solubilizer, such as alcohols (e.g., ethanol), polyalcohols (e.g., propylene glycol, polyethylene glycol), non-ionic surface active agents [e.g., Polysorbate 80, HCO-50 (polyoxyethylene (50 mol) adduct of hydrogenated castor oil)] and the like. The oily solution includes, for example, sesame oil and soybean oil, which may be employed in combination with a solubilizer, such as benzyl benzoate and benzyl alcohol. The injectable solutions thus prepared are normally filled into appropriate ampoules. The suppositories used for rectal administration are prepared by incorporating the compound (I) into conventional bases for suppositories.

The above-mentioned oral or parenteral pharmaceutical compositions are conveniently prepared in the unit dosage forms being adapted to correspond to the dose of the active ingredient. As such a unit dosage form, there may be illustrated tablets, pills, capsules, injectable solutions (ampoule), suppositories, etc., and

each of the respective unit dosage forms comprises normally 1 to 50 mg of the compound; among others, the injectable solution preferable contains 1 to 10 mg of the compound (I), while the other dosage forms preferably contain 1 to 25 mg of the compound.

In order to secure by far enhancement of the antitumor activities of the compound (I) and further reduction of its side effects, the compound (I) may be used as a lipid globule (e.g., emulsion, liposome) by adding natural phospholipids, such as yolk lecithin, soybean lecithin, sphingomyelin, phosphatidylserin, phosphatidylglycerol, phosphatidylinositol, diphosphatidylglycerol and phosphatidylethanolamine, or synthetic phospholipids, such as distearoylphosphatidylcholine and dipalmitoylphosphatidylethanolamine.

Each of the above-mentioned compositions may contain other active ingredients, unless their formulation with the compound (I) produces undesirable interactions.

The compounds (I) and their salts which are usable in the present invention can be produced by the method as described in the Japanese Unexamined Patent Publication No. 192825/1983 or those analogous thereto.

Illustrated below are production examples and examples to describe the present invention in more detail, whereby the compounds of Production Examples 3 and 5 are novel compounds.

Production Example 1

3-Octadecyl-2-pyrrolidinocarbonyl-1-tritylglycerol

In dichloromethane (10 mℓ) were dissolved 3-octadecyl-1-tritylglycerol (26 g) and pyridine (0.7 g), and phenyl chlorocarbonate (0.69 g) was added dropwise to the solution, followed by stirring at room temperature. After chloroform (25 mℓ) and water (25 mℓ) were added to the reaction solution, the mixture was stirred, and then the water layer was discarded. The organic layer was washed with 1 % aqueous sodium hydrogencarbonate solution, dried with sodium sulfate

and concentrated to dryness under reduced pressure. After pyrrolidine (5 mℓ) was added to the residue and the mixture was stirred at room temperature for 1 hour, then the reaction solution was concentrated to dryness under reduced pressure. The residue was purified by silica gel chromatography to give the objective compound (a colorless oily substance).

NMR (60 MHz, $CDCl_3$) δ: 0.88(3H,t,$CH_3$), 1.23(32H,s,-C$\underline{H}_2$-x16), 1.81(4H,m,C$\underline{H}_2$x2), 3.18 to 3.73(10H,m,OC$\underline{H}_2$x3, NC$\underline{H}_2$x2), 5.12(1H,quint,C$\underline{H}$-O$\overset{O}{C}$), 7.10 to 7.61(15H,m,aromatic protons)

IR (film) $cm^{-1}$: 2920, 2852, 1700, 1596, 1440, 1262, 1235, 1150, 1090, 760.

Production Example 2

3-Octadecyl-2-pyrrolidinocarbonylglycerol

In 80 % acetic acid (30 mℓ) was dissolved the trityl derivative (2.5 g) as obtained in Production Example 1, and the solution was heated at 110°C for 1.5 hours, with stirring. The reaction solution was concentrated to dryness under reduced pressure, and the residue was purified by silica gel chromatography (eluent; hexane-ethyl acetate (4:1)) to give the objective compound (a colorless oily substance) (1.3 g).

NMR (60 MHz, $CDCl_3$) δ: 0.88(3H,t,$CH_3$), 1.27(32H,s,-$CH_2$-x16), 1.81(4H,m,-$CH_2$-x2), 3.26 to 3.92(10H,m,O$CH_2$x3, NC$\underline{H}_2$x2), 4.89(1H,quint,C$\underline{H}$-O$\overset{O}{C}$).

IR (film) $cm^{-1}$: 3450, 2920, 2851, 1680, 1465, 1440, 1263, 1238, 1155, 1105, 1085, 755

Production Example 3

3-Octadecyloxy-2-(pyrrolidinocarbonyloxy)propyl 2-(N-methylpyrrolidinio)ethyl phosphate

In 10 mℓ of benzene was dissolved 1.3 g of the alcohol derivative as obtained in Production Example 2, and 1.07 g of bromoethyl phosphodichloridate and 0.45 g of triethylamine were added to the solution under icecooling, followed by stirring at room temperature for 2 hours. 10 mℓ of water and 0.45 g of triethylamine were added to the reaction solution, and the resulting mixture was stirred vigorously

for another 2 hours. The solvent was distilled off, and hydrochloric acid was added to the residue, followed by extraction with ether. The extract was washed with water, dried and concentrated to dryness. The residue was dissolved in 3 mℓ of N-methylpyrrolidine, and the solution was stirred at room temperature for 2 days and then concentrated to dryness. The residue was purified by silica gel chromatography ( eluent (1): methanol. (2): chloroform-methanol-water, 65:25:4) to give 0.89 g of the objective compound, a colorless solid.

NMR (60 MHz, $CDCl_3$) δ: 0.88(3H,t), 1.27(32H,brs), 1.81 (4H,m,pyrrolidino), 2.22(4H,m,pyrrolidinio), 3.23(3H, s,$\overset{+}{N}$Me), 3.2-4.1(16H,m,$CH_2O$x3, $NCH_2$x5), 4.30(2H,m,$POCH_2$), 4.96(1H,m,>CHOCO).

IR (KBr, $cm^{-1}$) : 3400, 2920, 2850, 1680, 1465, 1400, 1240, 1190, 1065.

## Production Example 4

3-Hexadecyl-2-pyrrolidinocarbonylglycerol

By the same procedure as described in Production Examples 1 and 2, a reaction was carried out to give a colorless, oily objective compound.

NMR (60 MHz, $CDCl_3$) δ: 0.88(3H,t), 1.25(28H,m), 1.80 (4H,m,$CH_2$x2), 3.2 to 3.95(10H,m,$OCH_2$x3, $NCH_2$x2), 4.89 (1H,m,>CHOCO)

IR (film, $cm^{-1}$): 3450, 2920, 2850, 1680, 1465, 1260, 1240, 1155, 1080.

## Production Example 5

3-Hexadecyloxy-2-(pyrrolidinocarbonyloxy)propyl 2-trimethylammonioethyl phosphate

In 10 mℓ of toluene containing 2 g of trimethylamine was dissolved the brominated derivative which was synthesized from the alcohol derivative (1.5 g) as obtained in Production Example 4, bromoethyl phosphodichloridate (1.30 g) and triethylamine (0.54 g) in the same manner as described in Production Example 3, and the solution was stirred at room temperature for 3 days. The reaction solution was

concentrated to dryness under reduced pressure, and the residue was purified by silica gel chromatography to give 1.15 g of the colorless, solid objective compound.

NMR (60 MHz, $CDCl_3$) δ: 0.88(3H,t), 1.25(28H,m,$CH_2$), 1.81 (4H,m,$CH_2$x2), 3.31(9H,s,$\overset{+}{N}Me_3$), 3.2 to 4.0(12H,$CH_2$Ox4, $NCH_2$x2), 4.30(2H,m,$POCH_2$), 4.94(1H,m,>CHOCO).

IR (KBr, $cm^{-1}$) : 3400, 2925, 2850, 1680, 1470, 1400, 1240, 1190, 1070.

## Example 1

### Tablet

Composition:

| | |
|---|---|
| (1) 1-O-Octadecyl-2-O-(morpholinocarbonyl)glycero-3-phosphocholine | 10 g |
| (2) Lactose | 85 g |
| (3) Corn starch | 20 g |
| (4) Hydroxypropylcellulose | 4 g |
| (5) Magnesium stearate | 1 g |
| | 120 g for 1000 tablets |

Preparation:

A mixture of (1), (2) and (3) is moistened with a 10 % aqueous solution of (4), and the resulting mixture is passed through a 1.5 mm screen and granulated, followed by vacuum drying at 40°C. The granules thus obtained are further screened and mixed with (5), and the mixture is compressed to prepare 1000 tablets of 7 mm in diameter each containing 10 mg of (1) per tablet.

## Example 2

### Sugar-coated tablet

The tablets as prepared in accordance with Example 1 are coated with shells consisting of sugar, talc and powdered gum arabic by the conventional method. After sugar coating, the coated tablets are polished with beeswax.

Weight of a sugar-coated tablet: 250 mg.

## Example 3

### Capsule

Composition:

| | |
|---|---|
| (1) 1-O-Octadecyl-2-O-(pyrrolidinocarbonyl)glycero-3-phosphocholine | 25 g |
| (2) Corn starch | 95 g |
| (3) Talc | 10 g |
| | 130 g for 1000 capsules |

Preparation:

All of the ingredients are blended and filled into 1000 gelatin capsules to prepare capsules which each contains 25 mg of (1).

Example 4

Injectable solution

In 1.0 ℓ of distilled water is dissolved 10 g of 3-octadecyloxy-2-(morpholinocarbonyloxy)propyl 2-(N-methylpyrrolidinio)ethyl phosphate, and after sterile filtration, the solution is distributed in 1 mℓ portions into 1000 vials under sterile conditions, lyophilized, followed by tight closing of the vials.

On the other hand, 2 ℓ of distilled water for injection containing 100 g of mannitol is distributed in 2 ml portions into ampoules for injection under sterile conditions, followed by fusion of the ampoules to prepare 1000 ampoules of injectable solution.

On the occasion of use, the powder contained in the former one vial is dissolved in the mannitol solution for injection.

The compound (I) represented by the formula (I), because of its potent antitumor activity and mild toxicity, can be used as an antitumor agent serving the useful purpose of producing the preventive, therapeutic and life-span-prolonging effects against tumors, such as leukemia and solid cancer.

For example, the compound exhibits excellent efficacy toward mice bearing the solid and ascites Sarcoma 180 (S-180) and Meth A tumors when administered intravenously, into the tumor tissues or intraperitoneally.

Also, the compound produces inhibitory effect in the *in vitro* proliferation inhibition test using myelogenous leukemia cells of a man(HL-60), while demonstrating marked immunologic enhancement action, particularly outstanding macrophage activating acitity. In addition, when the compound being usable in the present invention is administered intravenously or intraperitoneally to mice bearing the solid tumor such as the Meth A and S 180 tumors, necrosis through bleeding of the tumor tissue can be observed. Consequently, the anti-tumor effect of the compound being usable in the present invention is considered to be based on the complex effect having in combination the immunologic enhancement and direct cytocidal actions.

The test examples are described in the following to illustrate the effect of the present invention specifically.

Test Example 1

ICR mice (a group consisting of five mice) were inoculated intraperitoneally with 1 x $10^5$ S 180 cells per mouse, and then given 0.3 mg/mouse of 1-O-octadecyl-2-O-(morpholinocarbonyl)glycero-3-phosphocholine (Compound No. 1) dissolved in physiological saline once a day, three times in total, on the day of the inoculation and on the next day and the second day after the inoculation. The average of the survival days for the control group (consisting of 5 mice) was 12.2 days, whereas the number of surviving mice in the drug-treated group on the 50th day and the life-span prolongation ratio (T/C) against the control group, are shown in Table 1.

In a similar manner, a test was carried out with 1-O-octadecyl-2-O-methylglycero-3-phosphocholine used as a control drug (A), with the results being shown below:

Table 1:

Antitumor effect against mice bearing S 180 tumor.

| Test compound | Number of mice surviving for 50 days | T/C (%) |
|---|---|---|
| Compound 1 | 2 | >336 |
| Control drug A | 0 | 162 |

## Test Example 2

Activity toward mice bearing the Sarcoma 180 tumor.

ICR mice (male, 8-week-old, a group consisting of 5 mice), as divided into two groups, were transplanted subcutaneously with 1 x $10^6$ S 180 cells per mouse, respectively. One group was treated with the drug by injection into the tumor every four days commencing with the 7th day after the transplantation, four times in total. On the 24th day, the average tumor weight for the drug-treated group was measured, and the ratio (the tumor proliferation inhibition ratio) against that for the control group (not treated with the drug) was calculated. The results are shown in Table 2.

Table 2:

| Drug | Dose (mg/kg) | Weight of tumor (g) | Proliferation inhibition ratio (%) |
|---|---|---|---|
| Not treated (control group) | - | 1.49 | - |
| 2 | 25 | 0.71 | 52.4 |
| 3 | 25 | 0.39 | 73.9 |

Note:

Drug 2: 1-O-Octadecyl-2-O-(piperidinocarbonyl)glycero-3-phosphocholine

Drug 3: 3-Octadecyloxy-2-(morpholinocarbonyloxy)propyl 2-thiazolioethyl phosphate

## Test Example 3

Necrotic effect against the Meth A tumor tissue

Balb/c mice were transplated subcutaneously with 4 x $10^6$ Meth A cells, and 7 days later, treated with an aqueous solution of the drug (a solution in physiological saline) by intravenous injection, whereupon the tumor tissue was observed for any change. In the case where 1-O-octadecyl-2-O-(pyrrolidinocarbonyl)glycero-3-phosphocholine (25 mg/kg, given intravenously) and 1-O-octadecyl-2-O-(morpholinocarbonyl)glycero-3-phosphocholine (1.25 mg/kg, given intravenously) were employed as a drug,

the tumor tissue, initially appearing pink, turned red-black after 24 hours, and there was observed necrosis through bleeding. A similar test was carried out with ICR mice bearing the S 180 tumor, whereby similar changes of the tumor tissue were observed.

## Test Example 4

According to the method of R. Gallo et al. described in Blood 54 (3), 713 (1979), the growth inhibiting effect (IG effect) and differentiation inducing activity of 1-O-octadecyl-2-O-(morpholinocarbonyl)glycero-3-phosphocholine against human myelocytic leukemia cells HL-60 were assayed.

The results were that the IG effect on HL-60 ($GD_{50}$) was 0.6 μg/ml and that the compound had mild differentiation inducing activity at a concentration of 1-2 μg/ml.

## Test Example 5

Neither 1-O-octadecyl-2-O-(morpholinocarbonyl)glycero-3-phosphocholine nor 3-octadecyloxy-2-(morpholinocarbonyloxy)propyl 2-thiazolioethyl phosphate at a concentration of $3 \times 10^{-5}$ M caused platelet aggregation against rat platelet.

CLAIMS

1. Use of a compound of the formula

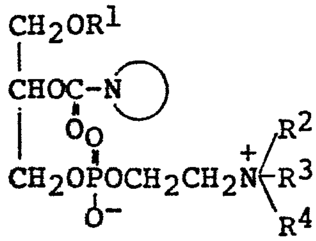

wherein $R^1$ is a $C_{14-19}$ alkyl group; $R^2$, $R^3$ and $R^4$ are independently $C_{1-2}$ alkyl or 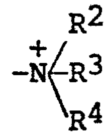 represents a nitrogen-containing five- or six-membered heterocyclic ring; and 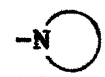 is a nitrogen-containing four- to six-membered heterocyclic group, or a pharmaceutically acceptable salt thereof, for the manufacture of a medicament for inhibiting the proliferation of tumors in a warm-blooded animal or prolonging the life span of said animal.

2. The use according to claim 1, wherein $R^1$ is a $C_{16-18}$ alkyl group.

3. The use according to claim 1, wherein $R^1$ is an octadecyl group.

4. The use according to claim 1, wherein $R^2$, $R^3$ and $R^4$ are methyl.

5. The use according to claim 1, wherein 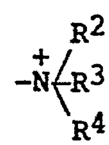 is thiazolio, oxazolio, N-methylpyrrolidinio, pyridinio, N-methylpiperazinio, N-methylmorpholinio or N-methylpiperidinio.

6. The use according to claim 1, wherein 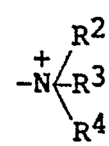 is thiazolio.

7. The use according to claim 1, wherein 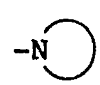 is azetidino, pyrrolidino, piperidino, morpholino, piperazino or thiomorpholino.

8. The use according to claim 1, wherein 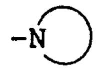 is pyrrolidino, piperidino, morpholino.

9. The use according to claim 1, wherein 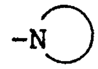 is morpholino.

10. The use according to claim 1, wherein the compound is 1-O-octadecyl-2-O-(morpholinocarbonyl)glycero-3-phosphocholine.

11. The use according to claim 1, wherein the compound is 1-O-octadecyl-2-O-(piperidinocarbonyl)glycero-3-phosphocholine.

12. The use according to claim 1, wherein the compound is 3-octadecyloxy-2-(morpholinocarbonyloxy)propyl 2-thiazolioethyl phosphate.

13. The use according to claim 1, wherein the compound is 3-octadecyloxy-2-(pyrrolidinocarbonyloxy)propyl 2-(N-methylpyrrolidinio)ethyl phosphate.

14. The use according to claim 1, wherein the compound is 3-hexadecyloxy-2-(pyrrolidinocarbonyloxy)propyl 2-trimethylammonioethyl phosphate.

0171968

European Patent Office

# EUROPEAN SEARCH REPORT

Application number

EP 85 30 5407

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,Y | JP-A-55 002 636 (TOYAMA CHEMICAL CO., LTD.) * Whole document, particularly page 238, compounds 3,13 * | 1-14 | A 61 K 31/685// C 07 F 9/65 |
| Y | EP-A-0 094 186 (TAKEDA CHEMICAL INDUSTRIES LTD.) * Page 1, line 1 - page 2, line 2; examples 36,37,45-47 * | 1-14 | |
| | | | TECHNICAL FIELDS SEARCHED (Int. Cl 4) |
| | | | A 61 K 31/00 C 07 F 9/00 |

The present search report has been drawn up for all claims

| Place of search | Date of completion of the search | Examiner |
|---|---|---|
| THE HAGUE | 11-11-1985 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82